(19) Europäisches Patentamt
European Patent Office
Office européen des brevets

(11) Veröffentlichungsnummer: **0 427 096 A1**

# (12) EUROPÄISCHE PATENTANMELDUNG

(21) Anmeldenummer: **90120853.8**

(22) Anmeldetag: **31.10.90**

(51) Int. Cl.5: **C07C 19/00**, C07C 17/42, B41M 5/165

(30) Priorität: **04.11.89 DE 3936731**

(43) Veröffentlichungstag der Anmeldung: **15.05.91 Patentblatt 91/20**

(84) Benannte Vertragsstaaten: **BE DE DK ES FR GB GR IT LU NL**

(71) Anmelder: **HOECHST AKTIENGESELLSCHAFT**
**Postfach 80 03 20**
**W-6230 Frankfurt am Main 80(DE)**

(72) Erfinder: **Landau, Helmut**
**Altvaterstrasse 4**
**W-8906 Gersthofen(DE)**

## (54) Verfahren zur Stabilisierung von chlorierten Paraffinen.

(57) Chlorierte Paraffine, welche aliphatische oder cycloaliphatische Amine als Stabilisatoren enthalten, eignen sich hervorragend als Lösemittel für die Farbbildner in chemisch reagierenden Durchschreibepapieren.

EP 0 427 096 A1

Xerox Copy Centre

## VERFAHREN ZUR STABILISIERUNG VON CHLORIERTEN PARAFFINEN

Die Erfindung bezieht sich auf die Stabilisierung von Chlorparaffinen, die als Lösemittel für die Farbbildner von chemisch reagierenden Durchschreibepapieren verwendet werden.

Die heute meist verwendeten kohlefreien Durchschreibepapiere arbeiten nach dem Prinzip der Farbbildung durch Reaktion einer elektronenabgebenden organischen Verbindung, dem sogenannten Farbbildner, welcher keine oder nur eine äußerst geringe Eigenfarbe besitzt, mit einer elektronenaufnehmenden Substanz, dem sogenannten Farbentwickler.

Die üblichen drucksensitiven Kopierpapiere bestehen aus einer Kombination eines Papieroberbogens, auf dessen Rückseite die mikroverkapselte Emulsion des Farbbildners aufgetragen ist (Geberschicht = CB-Schicht = Coated Back). Auf dem Papierunterbogen ist die elektronenaufnehmende Substanz für die Farbentwicklung aufgetragen (Nehmerschicht = CF-Schicht = Coated Front). Bei einem Durchschreibesatz mit 3 oder mehr Blättern wird mit Zwischenblättern gearbeitet, die auf der Oberseite die CF-Schicht und auf der Unterseite die CB-Schicht tragen (CFB-Zwischenblätter).

Durch Druck oder Schlag werden die Mikrokapseln, in welchen der gelöste Farbbildner eingeschlossen ist, zerstört und die Farbbildnerlösung sofort von der saugfähigen Nehmerschicht absorbiert und fixiert.

Als elektronenaufnehmende Substanzen oder Farbentwickler für die CF-Schicht sind bekannt:

- sauer aktivierte Aluminium-Schichtsilikate auf Montmorillonit-Basis, aktivierte Clay-Qualitäten, wie beispielsweise Silton, Bentonite, Attapulgite oder synthetische Produkte, wie beispielsweise behandelte Anhydrite, Aluminimsilikate, Magnesiumsilikate
- spezifische Zinksalicylat-Verbindungen
- phenolische Harze, wie z.B. Kondensationsprodukte von Paraphenylphenol, Para-Alkylphenolen mit Formaldehyd.

Als Farbbildner für die CB-Schicht werden beispielsweise Kristallviolettlacton, Malachitgrünlacton, Benzoylleucomethylenblau, Rhodamin-B-lacton u.ä. Substanzen verwendet. In den meisten Fällen wird ein Gemisch verschiedener Farbbildner eingesetzt.

Als Lösemittel für die Farbbildner können viele organische Flüssigkeiten verwendet werden, wobei die üblichen Substanzen modifizierte Terphenyle oder alkylierte Naphtaline oder Diphenyle in Kombination mit verschiedenen Kerosintypen sind.

Die Verwendung von geradkettigen chlorierten Paraffinkohlenwasserstoffen mit 6 bis 18 Kohlenstoffatomen und Chlorgehalten von 20 bis 60 Gew.-% als Lösemittel für die Farbbildner ist bekannt (vgl. GB 1 296 477). Chlorparaffine haben die gewünschten Eigenschaften, die an das Lösemittel gestellt werden. Sie sind gute Lösemittel für die Farbbildner, sie sind verfügbar in allen Viskositätsklassen durch entsprechende Variation der C-Kettenlängen der Paraffine und des Chlorierungsgrades. Sie besitzen keinen störenden Geruch. Chlorierte Paraffine verringern die Farbbildungsempfindlichkeit der CF-Schicht nicht.

Es hat sich jedoch gezeigt, daß eine Stabilisierung mit den üblicherweise in chlorierten Paraffinen eingesetzten epoxidierten Ölen z.B. epoxidiertem Sojabohnenöl oder epoxidgruppenhaltigen Harzen allein nicht ausreicht, eine Farbbildung der Farbbildner-Lösung selbst bei Raumtemperatur zu verhindern. Dieser Effekt macht sich durch eine Schleierbildung auf den Durchschlagpapieren bemerkbar, die durch erhöhte Temperatur oder bei Sonneneinstrahlung noch verstärkt wird. Auch die Intensität des Schriftbildes leidet unter dieser unerwünschten Farbbildung.

Die Aufgabe bestand daher darin, eine Stabilisierung zu finden, welche die vorstehend genannten Nachteile nicht aufweist.

Es wurde gefunden, das durch Zusatz bestimmter Amine zu dem chlorierten Paraffin die vorzeitige Verfärbung der Farbbildnerlösung verhindert werden kann.

Die Erfindung betrifft somit ein Verfahren zum Stabilisieren eines chlorierten, im wesentlichen geradkettigen Paraffins mit 7 bis 35 Kohlenstoffatomen und einem Chlorgehalt von 10 bis 72 Gew. -%, dadurch gekennzeichnet, daß man dem chlorierten Paraffin 0,05 bis 2 Gew.-%, bezogen auf das chlorierte Paraffin, mindestens eines Amins der Formel

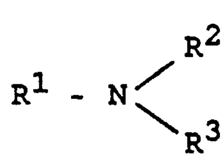 ,

worin $R^1$, $R^2$ und $R^3$ gleich oder verschieden sind und ein Wasserstoffatom, eine $C_3$-$C_{18}$-Alkylgruppe, eine $C_6$-$C_8$-Cycloalkylgruppe oder eine $C_2$-$C_6$-Aminoalkylgruppe bedeuten, wobei die Gesamtzahl der Kohlenstoffatome im Molekül mindestens sechs beträgt und der Siedepunkt bei Normaldruck oberhalb 90 °C liegt, zusetzt.

Die Erfindung betrifft weiterhin ein chloriertes, im wesentlichen geradkettiges Paraffin mit 7 bis 35 Kohlenstoffatomen und einem Chlorgehalt von 10 bis 72 Gew.-%, enthaltend 0,05 bis 2 Gew.-%, bezogen auf das chlorierte Paraffin, mindestens eines Amins der Formel

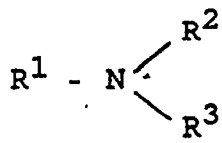 ,

worin $R^1$, $R^2$ und $R^3$ die vorgenannte Bedeutung haben, und seine Verwendung.

Die erfindungsgemäß zu stabilisierenden chlorierten Paraffine sind Chlorierungsprodukte von im wesentlichen geradkettigen Paraffinen mit 7 bis 35 Kohlenstoffatomen im Molekül, bevorzugt von technischen Paraffinschnitten mit beispielsweise 10 bis 13, 14 bis 17, 18 bis 24 und so weiter Kohlenstoffatomen. Der Chlorgehalt dieser Chlorierungsprodukte beträgt 10 bis ca. 72 Gew. -%.

Die erfindungsgemäß zu verwendenden Amine sind formal Derivate des Ammoniaks, in dem ein oder mehrere Wasserstoffatome durch Alkyl- oder Cycloalkylreste ersetzt sind. Je nach "Substitutionsgrad" wird danach zwischen primären, sekundären und tertiären Aminen unterschieden:

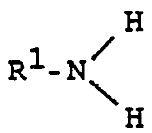 primäres Amin

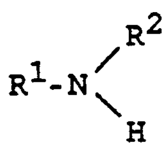 sekundäres Amin

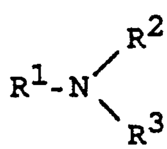 tertiäres Amin

$R^1$, $R^2$ und $R^3$ sind gleich oder verschieden und bedeuten ein Wasserstoffatom, eine $C_3$-$C_{18}$-, vorzugsweise $C_4$-bis $C_{12}$-Alkylgruppe, eine $C_6$-$C_8$-Cycloalkylgruppe oder eine $C_2$-$C_6$-Aminoalkylgruppe, wobei die Gesamtzahl der Kohlenstoffatome im Molekül mindestens sechs beträgt und der Siedepunkt bei Normaldruck oberhalb 90 °C liegt.

Die Alkylreste können geradkettig, verzweigtkettig oder cycloaliphatisch sein.

Ausgangsbasis für die Synthesen der Monoamine sind hauptsächlich die aus der OXO-Synthese (Hydroformierung) zur Verfügung stehenden Aldehyde bzw. Alkohole, die durch aminierende Hydrierung bzw. Ammonolyse (Austausch der OH-Gruppen im Alkohol durch Ammoniak) in die entsprechenden Amine überführt werden.

Die cyclischen Amine entstehen durch Umsetzung von Aldehyden und Ketonen mit Ammoniak bzw. primären Aminen.

Neben den Monoaminen ist auch die Verwendung von Di- und Triaminen für die Stabilisierung der oben beschriebenen Chlorparaffine möglich. Die Herstellung der Di- und Triamine verläuft vorwiegend über Additionsreaktion an Acrylnitril. Die zunächst entstehenden Aminonitrile werden durch nachgeschaltete katalytische Hydrierung in die entsprechenden Di- und Triamine umgewandelt.

Geeignete Amine sind beispielsweise Di-n-propylamin, Tri-n-propylamin, n-Amylamin, n-Hexylamin, Octylamin, Di-n-octylamin, Tri-n-octylamin, Di-isononylamin, Di-(2-ethylhexyl)amin, Ethylendiamin, Laurylamin.

Die Alkylamine oder Cycloalkylamine sind im chlorierten Paraffin in einer Menge von 0,05 bis 2 Gew.-%, vorzugsweise 0,05 bis 1,8 Gew.-%, bezogen auf das chlorierte Paraffin, enthalten.

Das verwendete chlorierte Paraffin kann zusätzlich auch die bekannten Stabilisatoren, beispielsweise epoxidierte Öle, wie epoxidiertes Sojabohnenöl, oder epoxidierte Harze, beispielsweise cycloaliphatisches Diepoxid, in einer Menge von 1 bis 4 Gew.-% enthalten. Möglich ist auch eine Stabilisierung mit einer Kombination cycloaliphatischer Glycidylester mit tertiären Estern der phosphorigen Säure.

Die Stabilisierung des chlorierten Paraffins erfolgt zweckmäßigerweise unmittelbar nach beendeter Chlorierung durch Einarbeitung des Stabilisators oder der Stabilisatoren in das noch warme Chlorierungsprodukt. Man erreicht auf diese Weise optimale Stabilitätsgrade.

Derartig stabilisierte chlorierte Paraffine ergeben als Lösemittel für die Farbbildner keine Schleierbildung der damit hergestellten Durchschlagpapiere. Infolge der guten Löslichkeit der Alkylamine in chlorierten Paraffinen sind zusätzliche Solubilisierungsmittel nicht erforderlich.

Die erfindungsgemäß stabilisierten chlorierten Paraffine können, wie in der Praxis meist üblich, mit einem anderen Lösemittel, beispielsweise mit einem Kohlenwasserstoff-Verdünnungsmittel, zum Beispiel einem Kerosintyp, vermischt werden.

Die Erfindung wird in den nachfolgenden Beispielen näher erläutert.

**Beispiel 1:**

In je 100 g mit einem Alkylamin stabilisierten chlorierten Paraffin wurde 0,2 g Farbbildner (CVL = Kristallviolettlacton) gelöst. Diese Lösung wurde über einen Zeitraum von 80 min auf 120 °C erhitzt. In Zeitabständen von 10, 15, 20, 30, 40, 50, 60 und 80 min wurden Proben entnommen, schnell auf Raumtemperatur gekühlt und die optische Dichte (Lichtabsorption) der Probe bei 605 nm in einer 1 cm-Küvette mit einem Spektralphotometer gemessen. Als Blindprobe diente das gleiche chlorierte Paraffin mit Alkylamin, aber ohne Farbbildner, das unter gleichen Bedingungen erhitzt worden war. Am Ende der Versuchszeit von 80 min wurde außerdem die Jodfarbzahl der Farbbildnerlösung zur Feststellung der Alterung des chlorierten Paraffins gemessen.

**Beispiel 2:**

In je 100 g mit einem Alkylamin stabilisierten chlorierten Paraffin wurde 0,2 g Farbbildner (BLMB = Benzoyllekomeuthylenblau) gelöst. Diese Lösung wurde über einen Zeitraum von 80 min auf 120 °C erhitzt. In Zeitabständen von 10, 15, 20, 30, 40, 50, 60 und 80 min wurden Proben entnommen, schnell auf Raumtemperatur gekühlt und die optische Dichte der Probe bei 660 nm in einer 1 cm-Küvette mit einem Spektralphotometer gemessen. Als Blindprobe diente das gleiche chlorierte Paraffin mit Alkylamin, aber ohne Farbbildner, das unter gleichen Bedingungen erhitzt worden war. Am Ende der Versuchszeit von 80 min wurde außerdem die Jodfarbzahl der Farbbildnerlösung zur Feststellung der Alterung des chlorierten Paraffins gemessen.

**Beispiel 3:**

In je 100 g mit einem Alkylamin stabilisierten chlorierten Paraffin wurde 0,2 g Farbbildner (Farbstoffgemisch, bestehend aus 50 Gew.-% Kristallviolettlacton, 15 Gew.-% Benzoylleukomethylenblau,15 Gew.-% Olive 1-G und 20 Gew.-% Rot 1-6B) gelöst. Bei den Farbstoffen handelt es sich um Handelsprodukte. Diese Lösung wurde über einen Zeitraum von 80 min auf 120 °C erhitzt. In Zeitabständen von 10, 15, 20, 30, 40, 50, 60 und 80 min wurden Proben entnommen, schnell auf Raumtemperatur gekühlt und die optische Dichte der Probe bei 605 nm in einer 1 cm-Küvette mit einem Spektralphotometer gemessen. Als Blindprobe diente das gleiche chlorierte Paraffin mit Alkylamin, aber ohne Farbbildner, das unter gleichen Bedingungen erhitzt worden war. Am Ende der Versuchszeit von 80 min wurde außerdem die Jodfarbzahl der Farbbildnerlösung zur Feststellung der Alterung des chlorierten Paraffins gemessen.

Eingesetzt wurden folgende chlorierte Paraffine:

A: chloriertes, im wesentlichen geradkettiges Paraffin mit 14-17 Kohlenstoffatomen und einem Chlorgehalt von ca. 40 Gew.-%, Handelsprodukt.

B: chloriertes, im wesentlichen geradkettiges Paraffin mit 10-13 Kohlenstoffatomen und einem Chlorgehalt von ca. 40 Gew.-%, Handelsprodukt.

Die chlorierten Paraffine waren mit einem cycloaliphatischen Diepoxid (Handelsprodukt) stabilisiert.

Tabelle 1

| chlorierter Paraffintyp: A 1 % epoxidiertes Harz 0,2 % Farbstoff CVL Alkylamin: 1,5 % | | | | | | | | | | |
|---|---|---|---|---|---|---|---|---|---|---|
| Tri-n-octyl- | Alkylamin Di-isononyl- | Di-(2-ethylhexyl)- | optische Dichte in % Absorption nach | | | | | | | Jodfarbzahl |
| | | | 10 | 15 | 20 | 30 | 40 | 60 | 80 | |
| | | | min | | | | | | | |
| - | - | - | 7 | 23 | 37 | 48 | 62 | 70 | 75 | 15 |
| + | - | - | 0 | 0 | 0 | 0 | 1 | 2 | 3 | 15 |
| - | - | + | 0 | 0 | 0 | 0 | 1 | 1 | 2 | 10 |

Tabelle 2

| chlorierter Paraffintyp: B 1 % epoxidiertes Harz 0,2 % Farbstoff CVL Alkylamin: 1,5 % | | | | | | | | | | |
|---|---|---|---|---|---|---|---|---|---|---|
| Alkylamin Tri-n-octyl- | Di-isononyl- | Di-(2-ethylhexyl)- | optische Dichte in % Absorption nach | | | | | | | Jodfarbzahl |
| | | | 10 | 15 | 20 | 30 | 40 | 60 | 80 | |
| | | | min | | | | | | | |
| - | - | - | 10 | 30 | 42 | 60 | 73 | 80 | 85 | 10 |
| + | - | - | 0 | 0 | 1 | 1 | 2 | 2 | 2 | 5 |
| - | + | - | 0 | 0 | 0 | 0 | 0 | 1 | 2 | 5 |
| - | - | + | 0 | 0 | 0 | 0 | 1 | 1 | 1 | 5 |

Tabelle 3

| chlorierter Paraffintyp: B 1 % epoxidiertes Harz 0,2 % Farbstoff BLMB Alkylamin: 1,5 % | | | | | | | | | | |
|---|---|---|---|---|---|---|---|---|---|---|
| Alkylamin Tri-n-octyl- | Di-isononyl- | Di-(2-ethylhexyl)- | optische Dichte in % Absorption nach | | | | | | | Jodfarbzahl |
| | | | 10 | 15 | 20 | 30 | 40 | 60 | 80 | |
| | | | min | | | | | | | |
| - | - | - | 3 | 10 | 15 | 26 | 32 | 41 | 52 | 5 |
| + | - | - | 0 | 0 | 0 | 1 | 1 | 2 | 3 | 5 |
| - | + | - | 0 | 0 | 0 | 0 | 0 | 1 | 1 | 5 |
| - | - | + | 0 | 0 | 0 | 0 | 0 | 0 | 1 | 5 |

Tabelle 4

| chlorierter Paraffintyp: B 1 % epoxidiertes Harz 0,2 % Farbstoffgemisch Alkylamin: 1,5 % | | | | | | | | | | |
|---|---|---|---|---|---|---|---|---|---|---|
| Alkylamin Tri-n-octyl- | Di-isononyl- | Di-(2-ethylhexyl)- | optische Dichte in % Absorption nach | | | | | | | Jodfarbzahl |
| | | | 10 | 15 | 20 | 30 | 40 | 60 | 80 | |
| | | | min | | | | | | | |
| - | - | - | 20 | 40 | 80 | 100 | | | | 10 |
| + | - | - | 0 | 0 | 0 | 1 | 1 | 2 | 2 | 10 |
| - | + | - | 0 | 0 | 0 | 0 | 0 | 1 | 1 | 5 |
| - | - | + | 0 | 0 | 0 | 0 | 0 | 0 | 1 | 5 |

## Ansprüche

1. Verfahren zum Stabilisieren eines chlorierten, im wesentlichen geradkettigen Paraffins mit 7 bis 35 Kohlenstoffatomen und einem Chlorgehalt von 10 bis 72 Gew.-%, dadurch gekennzeichnet, daß man dem chlorierten Paraffin 0,05 bis 2 Gew.-%, bezogen auf das chlorierte Paraffin, mindestens eines Amins der Formel

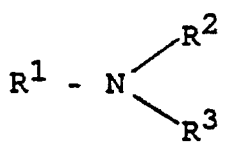 ,

worin $R^1$, $R^2$ und $R^3$ gleich oder verschieden sind und ein Wasserstoffatom, eine $C_3$-$C_{18}$-Alkylgruppe, eine $C_6$-$C_8$-Cycloalkylgruppe oder eine $C_2$-$C_6$-Aminoalkylgruppe bedeuten, wobei die Gesamtzahl der Kohlenstoffatome im Molekül mindestens sechs beträgt und der Siedepunkt bei Normaldruck oberhalb 90 °C liegt, zusetzt.

2. Verfahren nach Anspruch 1, dadurch gekennzeichnet, daß das Amin ein primäres, sekundäres oder tertiäres Alkylmonoamin oder ein Polyamin ist.

3. Verfahren nach Anspruch 1, dadurch gekennzeichnet, daß dem chlorierten Paraffin zusätzlich 0,05 bis 4 Gew.-%, bezogen auf das chlorierte Paraffin, eines Epoxidstabilisators zugesetzt wird.

4. Chloriertes, im wesentlichen geradkettiges Paraffin mit 7 bis 35 Kohlenstoffatomen und einem Chlorgehalt von 10 bis 72 Gew.-%, enthaltend 0,05 bis 2 Gew.-%, bezogen auf das chlorierte Paraffin, mindestens eines Amins der Formel

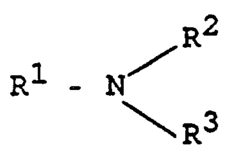 ,

worin $R^1$, $R^2$ und $R^3$ gleich oder verschieden sind und ein Wasserstoffatom, eine $C_3$-$C_{18}$-Alkylgruppe, eine $C_6$-$C_8$-Cycloalkylgruppe oder eine $C_2$-$C_6$-Aminoalkylgruppe bedeuten, wobei die Gesamtzahl der Kohlenstoffatome im Molekül mindestens sechs beträgt und der Siedepunkt bei Normaldruck oberhalb 90 °C liegt.

5. Verwendung des chlorierten Paraffins nach Anspruch 1 zur Herstellung chemisch reagierender Durchschreibepapiere.

Europäisches Patentamt

# EUROPÄISCHER RECHERCHENBERICHT

Nummer der Anmeldung

**EP 90 12 0853**

## EINSCHLÄGIGE DOKUMENTE

| Kategorie | Kennzeichnung des Dokuments mit Angabe, soweit erforderlich, der maßgeblichen Teile | Betrifft Anspruch |
|---|---|---|
| X | DE-C-6 851 25 (J.K. WIRTH)<br>* Insgesamt *<br>– – – | 1-2,4 |
| Y | | 5 |
| X | EP-A-0 023 540 (HOECHST)<br>* Patentansprüche *<br>– – – | 1-2,4 |
| Y | | 5 |
| X | FR-A-1 505 678 (WACKER-CHEMIE)<br>* Résumé *<br>– – – | 1-2,4 |
| Y | | 5 |
| X | US-A-2 543 575 (C.C. HARVEY et al.)<br>* Patentansprüche *<br>– – – | 1-2,4 |
| Y | | 5 |
| Y | DE-C-3 534 984 (HOECHST)<br>* Patentansprüche *<br>– – – | 5 |
| X | US-A-4 032 584 (IRANI)<br>* Patentansprüche *<br>– – – – – | 3 |

**KLASSIFIKATION DER ANMELDUNG (Int. Cl.5)**

C 07
C 19/00
C 07 C 17/42
B 41 M 5/165

**RECHERCHIERTE SACHGEBIETE (Int. Cl.5)**

C 07 C 17/00
B 41 M 5/00

Der vorliegende Recherchenbericht wurde für alle Patentansprüche erstellt

| Recherchenort | Abschlußdatum der Recherche | Prüfer |
|---|---|---|
| Den Haag | 04 Februar 91 | VAN GEYT J.J.A. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument